# EUROPÄISCHE PATENTANMELDUNG

(11) **EP 2 295 114 A1**
(43) Veröffentlichungstag der Anmeldung: **16.03.2011**
(21) Anmeldenummer: 09011583.3
(22) Anmeldetag: 10.09.2009
(51) Int. Cl.: A61Q 15/00, A61K 8/34, A61K 8/49

(54) **Kosmetische Zusammensetzung mit antimikrobieller Wirkung**

(71) Anmelder: Dalli-Werke GmbH & Co. KG, 52224 Stolberg (DE)
(72) Erfinder: Bonn, Andrea, 52372 Kreuzau (DE); Esser, Christoph, 52146 Würselen (DE); Gilliam, Herman, 6369 BH Simpelveld (NL); Krichel, Jürgen., 52428 Jülich (DE)
(74) Vertreter: Polypatent

(57) **Zusammenfassung**

Die vorliegende Erfindung betrifft eine kosmetische Zusammensetzung, die in Form eines homogenen Gemisches vorliegt, wobei das Gemisch neben einem antimikrobiellen Wirksystem einen besonders hohen Anteil an ÖI-/Lipidphase aufweist.

## Beschreibung

Die vorliegende Erfindung betrifft eine kosmetische Zusammensetzung, die in Form eines homogenen Gemisches vorliegt, wobei das Gemisch neben einem antimikrobiellen Wirksystem einen besonders hohen Anteil an Öl-/Lipidphase aufweist.

Kosmetische Zusammensetzungen, die pflegende Substanzen und weitere Wirkstoffe enthalten, liegen in den häufigsten Fällen als Emulsionen, Dispersionen oder Suspensionen vor. Vor allem Artikel zur Hautpflege werden häufig als Öl-in-Wasser- (O/W) oder Wasser-in-Öl-Emulsionen (W/O) angeboten, wobei die verschiedenen pflegenden und eine andere Funktion erfüllenden Wirkstoffe in den unterschiedlichen Phasen gelöst oder dispergiert vorliegen können.

Körpergerüche entstehen durch die Einwirkung von Hautbakterien auf apokrinen Schweiß, wobei unangenehm riechende Abbauprodukte gebildet werden. Eine Gruppe der kosmetischen Zusammensetzungen mit Wirkstoffen, die nicht nur der Hautpflege, sondern vor allem einer anderen Funktion dienen, sind die Deodorants, bzw. die Antitranspirants. In diesen Zusammensetzungen sind entweder desodorierende Wirkstoffe enthalten, also Wirkstoffe, die einer Entwicklung von unangenehmen Körpergerüchen entgegenwirken sollen, oder Antitranspirantien, also Wirkstoffe, die die Bildung oder die Ausscheidung von Schweiß verringern (astringente Wirkstoffe).

Üblicherweise werden Deodorants, die (einen) desodorierende(n) Wirkstoff(e) enthalten, in erster Linie als alkoholische Lösung, oder als Emulsion bereitgestellt. Solche Deodorants auf Alkoholbasie haben allerdings den Nachteil, dass die Haut in den Bereichen, auf die das Deodorant aufgetragen wird, leicht austrocknet und es zu einer unangenehmen Hautreaktion wie Brennen oder Jucken kommen kann.

Antitranspirants werden dagegen häufig als eine Dispersion eines antitranspiranten Wirkstoffes, üblicherweise eines Aluminiumsalzes, in einer Ölphase oder in Form einer O/W- oder einer W/O-Emulsion bereitgestellt. Bei solchen Zusammensetzungen kommt es in den meisten Fällen zur Bildung weißer Flecken oder Ränder auf Haut und Kleidung In dem Bereich, in dem die Zusammensetzung aufgetragen wurde, da das Salz sich nach Einziehen der Ölphase auf Haut und Kleidung niederschlägt.

Aufgabe der vorliegenden Erfindung war es eine kosmetische Zusammensetzung bereitzustellen, die der Entwicklung eines unangenehmen Körpergeruches durch Schwitzen entgegenwirkt, ein angenehmes Hautgefühl ergibt, die Haut nicht austrocknet und gute Pflegeeigenschaften aufweist. Darüber hinaus sollte die Bildung von weißen Rändern und Flecken auf der Haut und der Kleidung vermieden werden.

Diese Aufgabe wird gelöst durch eine kosmetische Zusammensetzung in Form eines homogenen Gemisches, umfassend
(a) 0,01 - 30 % ein antimikrobielles Wirksystem,
(b) 0 - 70 % ein Lösungsmittelsystem für (a) und/oder (c) und/oder (d)
(c) optional wenigstens einen üblichen Hilfs- oder Zusatzstoff, soweit sie in (a), (b) und/ oder (c) löslich oder mit diesen mischbar sind, und
(d) ad 100% ein Öl-/ Lipidsystem.

Die erfindungsgemäße Zusammensetzung zeichnet sich dadurch aus, dass sie beim Aufbringen auf die Haut eine hohe Pflegewirkung erzielt und der Entwicklung von unangenehmem Körpergeruch entgegenwirkt.

Ein weiteres Merkmal der Zusammensetzung ist es, dass sämtliche Bestandteile der Zusammensetzung miteinander mischbar sind oder ein Bestandteil in wenigstens einem der anderen Bestandteile lösbar ist, so dass sich insgesamt ein einphasiges, bevorzugt klares bis opaleszentes, homogenes Gemisch ergibt.

Anitmikrobielles Wirksystem (a):
Das antimikrobielle Wirksystem (a) umfasst Substanzen, die auf die Bakterien, die zur Entwicklung des unangenehmen Geruches durch Abbau des Schweißes beitragen, eine keimhemmende, bakteriostatische oder eine keimtötende, bakterizide Wirkung haben (beide Wirkungen werden hier als "antibakterielle Wirkung" zusammengefasst).

Eine weitere Gruppe geeigneter antimikrobieller Wirkstoffe sind Substanzen, die auf mikrobielle Enzyme, die an der Umsetzung des Schweißes zu unangenehm rlechenden Abbauprodukten beteiligt sind, als Inhibitoren wirken.

Bevorzugt sind die antimikrobiellen Substanzen in organischen Lösungsmittein, insbesondere in Lösungsmitteln gemäß (b) oder (d) löslich, oder sie stellen selbst eine organische Flüssigkeit bzw. eine Flüssigkeit, die mit (b) oder (d) homogen mischbar ist, dar.

Als antibakterielle Substanzen sind grundsätzlich alle gegen grampositive Bakterien wirksamen Stoffe geeignet, wie z. B. 4-Hydroxybenzoesaeure und ihre Salze und Ester, N-(4- Chlorphenyl)-N'-(3,4-dichlorphenyl)hamstoff, 2,4,4'-Trichlor-2'-hydroxydiphenylether (Triclosan), 4- Chlor-3,5-dimethylphenol, 2,2-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-lod-2-propinylbutylcarbamat, Chlorhexidin, 3.4,4'-Trichlorcarbonilid (TTC), antibakterielle Riechstoffe, Thymol, Thymianoel, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsaeure-N-alkylamide wie z. B. Salicylsaeure-n- octylamid oder Salicylsaeure-n-decylamid. Diese Substanzen stellen eine gemäß der Erfindung bevorzugte Gruppe von antimikrobiellen Wirkstoffen dar.

Weitere typische Beispiele für geeignete antibakterielle Wirkstoffe sind Phenoxyethanol oder Ethylhexylglycerin. Als ebenfalls sehr wirkungsvoll hat sich auch 5-Chlor-2-(2,4-dichlorphenoxy)-phenol erwiesen, das unter der Marke Irgasan^{™} von der Firma Ciba, Basel/CH vertrieben wird. Diese Substanzen stellen eine weitere gemäß der Erfindung bevorzugte Gruppe von antimikrobiellen Wirkstoffen dar.

Auch Parabenverbindungen können erfindungsgemäß eingesetzt werden. Parabenverbindungen schließen insbesondere ein : Methylparaben (CAS 99-76-3), Ethyl-paraben (CAS 120-47-8), Propylparaben (CAS 94-13-3), Butylparaben (CAS 94-26-8), Isopropylparaben (CAS 4191-73-5), and Benzylparaben (CAS 94-18-8). Parabene sind eine weitere gemäß der Erfindung bevorzugte Gruppe von antimikrobiellen Substanzen.

Ebenfalls wirksam gemäß der Erfindung einsetzbar sind Isothiazoline, insbesondere Methyl-4-isothiazolin, Benzisothiazolin-on oder Benzchlorisothiazolin-on, beispielsweise erhältlich unter dem Namen Acticide MBS der Firma Thor.

Eine weitere gemäß der Erfindung bevorzugte Gruppe von antimikrobiellen Substanzen sind in organischen Lösungsmitteln lösliche Silber- oder Zinkverbindungen, wie beispielsweise Zinkphenolsulfonat, Zinkoxide, Zinkchloride, Zinkundecylenat, und Mischungen davon, oder auch weitere Zinkverbindungen, ausgewählt aus den unten genannten.

Eine besonders bevorzugte Gruppe von antimikrobiellen Wirkstoffen ist Ethylhexylglycerin, Phenoxyehtanol, Wirkstoffe aus der Gruppe der Parabene oder der Isothiazoline, Triclosan, und die oben genannten Zinkverbindungen. Aus dieser Gruppe wird bevorzugt wenigstens ein Wirkstoff, weiter bevorzugt eine Kombination von wenigstens zwei Wirkstoffen in die erfindungsgemäße Zusammensetzung eingearbeitet. Eine besonders bevorzugte Kombination ist Ethylhexylglycerin mit Phenoxyehtanol, wobei zusätzlich ein weiterer Wirkstoff verwendet werden kann, aber nicht muß.

Weitere antimikrobielle Wirkstoffe, die in die erfindungsgemäße Zusammensetzung einsetzbar sind, sind die antimikrobiellen Substanzen, die in folgenden Schriften genannt sind, soweit sie selbst eine organische bzw. eine mit (b) und/ oder (d) homogen mischbare Flüssigkeit darstellen oder in einem organischen Lösungsmittel löslich sind und soweit sie gut hautverträglich sind: US 5,500,217, US 6,001,341, US 6,060,043, EP 1 029 530 A1, US 2005/239903 und EP 2 082 724 A1 und WO 00/28956. Dies sind im Einzelnen:
Cetyl-trimethylammoniumbromid, Cetyl-pyridiniumchlorid, Benzethoniumchlorld, Diisobutyl phenoxy ethoxy ethyl dimethyl benzyl ammoniumchlorid, Natrium-N-laurylsarcosine, Natrium-N-palmethyl-sarcosine, Lauroylsarcosin, N-myristoylglycin, Kalium-N-lauryl-sarcosin, Trimethylammoniumchlorid, Natriumaluminumchlorhydroxylactat, Triethylcitrat, Tricetylmethylammoniumchlorid, 2,4,4'-trichlor-2'-hydroxydiphenylether (Triclosan), 3,4,4'-trichlorcarbanilid bzw. N-(4 chlorphenyl)-N'-(3,4-dichlorphenyl)-Harnstoff (Triclocarban), Diaminoalkylamide wie L-lysinhexadecylamid, Schwermetallsalze von Citrat, Salicylat, und Piroctose, insbesondere Zinksalze, und Säuren davon, Schwermetallsalze von Pyrithion, insbesondere Zinkpyrithion, Zinkphenolsulfat, Famesol, und Kombinationen davon. Auch Silberhalogenide, Octoxyglycerin (beispielsweise SENSIVA^{™} SC 50) und verschiedene Zinksalze, z.B. Zinkricinoleat weisen eine geeignete antimikrobielle Wirkung auf.

Ebenfalls geeignete Wirkstoffe, soweit sie in organischen Lösungsmitteln, insbesondere in (b) oder (d) löslich sind oder selbst organische Flüssigkeiten darstellen, sind: Alkyldimethylbenzalkoniumchlorid, 3-Acetyl-6-methylpyran-2,4 (3H)-dion, Benzalkoniumchlorid, Benzethoniumchlorid, -bromide and -saccharinat, Benzylalkohol, Chlorhexidin, Chlorbutanol, Chlorbutanol-4-hydroxybenzoesäure und deren Salze und Ester, Chlorxylenol, Chloflucarban, Cloquinol, Cocamidopropyl PGdiammoniumchloridphosphat. Ethanol, Fluorosalan, Hexachlorophen, Hexylresorcinol, Imidhamstoff, Isopropylalkohol, Mercufenolchlorid, Methylbenzethoniumchloride, Metronidazol, Nonylphenoxypoly(ethyleneoxy)-ethanoliodin, Octoxyglycerin, Phenol, Poloxamer-lod Komplex, Polyhexamethylenbuguanid (PHMB), Providoiondin, Quartäre Ammoniumverbindungen (QACs), Sekundäre Amyltricresole, Natriumbenzoat, Natriumoxychlorsen, Tribromsalan, Undecoylium-Chlorid-lod Komplex, Usninsäure und Zinkphenolsulfonat.

Auch folgende Wirkstoffe mit antimikrobieller Wirkung sind aus der Literatur bekannt und können in die erfindungsgemäße Zusammensetzung eingesetzt werden, soweit sie selbst eine organische Flüssigkeit darstellen oder in einem organischen bzw. einem unpolaren Lösungsmittel löslich sind und soweit sie hautverträglich sind: 3,3'-Dibromo-4,4'-hexamethylendioxydibenzamidin (Dibromhexamidin) und dessen Salze (einschließlich isethionate), Thiomersal, Phenylmercursäuresalze (einschließlich Borat), Hexetidine, 5-Brom-5-nitro-1,3-dioxan, 2-bromo-2-nitropropane-1,3-diol (Bronopol), 2,4-Dichlorbenzylalkohol, 4-Chlor-m-cresol, 4-Chlor-3,5-xylenol, 3,3'-Bis (1-hydroxymethyl-2,5-dioxoimidazolidin-4-yl)-1,1'-methylendiharnstoff, Hexamethylentetramin(methenamin), Methenamin 3-chlorallylchlorid, 1-(4-Chlorohenoxy)-1-(imidazol-1 -yl)-3,3-dimethylbutan-2-one, 1,3-Bis (hydroxymethyl)-5,5-dimethylimidazolidin-2,4-dion, 1-Hydroxy-4-methyl-6 (2,4,4-trimethylpentyl) 2-pyridon und dessen Monoethanolaminsalze, 1,2-Dibrom-2,4-dicyanobutan (Methyldibromglutaronitril), 6,6-Dibrom-4,4-dichlor-2,2'-methylen-diphenol (Bromchlorophen), eine Mischung aus 5-Chlor-2-methyl-isothiazol-3 (2H)-on and 2-methylisothiazol-3(2H)-on mit Magnesiumchlorid und Magnesiumnitrat, 2-Benzyl-4-chlorphenol (Chlorophen), 2-Chloracetamid, Chlorhexidin und dessen Digluconat, Diacetat und Dihydrochlorid, Alkyl (C₁₂-C₂₂) trimethylammoniumbromid und -chlorid, 4,4-Dimethyl-1,3-oxizalidin, N-(Hydroxymethyl)-N-(dihydroxymethyl-1,3-dioxo-2,5-imidazolidinyl-4)-N'-(hydroxymethyl)hamstoff, 1,6-Di (4-amidinophenoxy)-n-hexan (Hexamidin) und dessen Salze (einschließlich Isethionat and p-Hydroxybenzoat), Glutaraldehyd (Pentane-1,5-dial), 5-Ethyl-3,7-dioxa- -azabicyclo [3.3.0]octan 3-(p-chlorophenoxy)-propane-1,2 diol (Chlophihenesin), Benzylhemiformal, 3-lodo-2-propynylbutylcarbamat, Methylisothiazolinon (INCI) und Bezaldehyd und dessen Derivate 4-Methylbenzaldehyde, Heliotropin, Vanillin, 4-Hydroxybenzaldehyd, 3-Hydroxybenzaldehyd, 4-Methoxybenzaldehyd, 3-Methoxybenzaldehyd, 3-Hydroxy-4-methoxybenzaldehyd (Isovanillin), Borsäure, quatäre Ammoniumsalze, Benzyldimethylalkylammoniumchloride, in denen die Alkylgruppe 8 bis 18 Kohlenstoffatome enthält, 8-Hydroxychinolinsulfat, Hexachlorophene, Bithionol verschiedene Antibiotica und optisch aktive Aminosäuren, insbesondere D-Aminosäuren, wie Alanin Analoge, z.B., D-Cycloserin und 3-Chloro-D-Alanin, Aminosäuren, die ein Halogen in einer Seitenkette tragen, Methyl-Amino acids der Formel NH₂-C (CH₃) (COOH)-R wobei R ausgewählt ist aus Wasserstoff, Phenyl, und C₁-C₈ Alkyl , substituiert mit Phenyl, Hydroxy, Carboxy, Benzyloxy, Benzyloxycarbonyl, Halogen, und Amino, wobei das Halogen F, Cl, Br, und I, sein kann.

### Esteraseinhibitoren als antimikrobielle Substanzen

Beim Vorhandensein von Schweiß im Achselbereich werden durch Bakterien extrazelluläre Enzyme wie Esterasen, vorzugsweise Proteasen und/oder Lipasen - gebildet, die im Schweiß enthaltene Ester spalten und dadurch Geruchsstoffe freisetzen. Hiergegen wirken Esteraseinhibitoren als antimikrobielle Komponente, vorzugsweise Trialkylcitrate wie Trimethylcitrat, Tripropylcitrat, Truesopropylcitrat, Tributylcitrat und insbesondere Triethylcitrat (Hydagen^{™} CAT, Cognis GmbH, Duesseldorf/FRG). Die Stoffe inhibieren die Enzymaktivität und reduzieren dadurch die Geruchsbildung. Eine Hypothese ist, dass dabei durch die Spaltung des Zitronensäureesters die freie Säure freigesetzt wird, die den pH-Wert auf der Haut soweit absenkt, dass dadurch die Enzyme durch Acylierung inaktiviert werden. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw. -phosphat, Dicarbonsaeuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Zitronensäure, Äpfelsäure, Weinsäure oder Weinsäurediethylester sowie Zinkglycinat.

Das antimikrobielle Wirksystem stellt 0,01 bis 30 Gew% der gesamten kosmetischen Zusammensetzung dar, bevorzugt 1 bis 25 Gew%, weiter bevorzugt 3 bis 20 Gew%. Das Wirksystem kann aus einer der oben genannten antimikrobiellen Substanzen bestehen, oder aus zwei, drei oder mehreren der Substanzen zusammengesetzt sein, wobei im letzteren Fall bevorzugt von jeder Substanz wenigstens 0,01 Gew% in der kosmetischen Zusammensetzung enthalten ist. Bei einer Kombination verschiedener Substanzen summieren sich deren Einsatzkonzentrationen auf den oben genannten Gesamtwert von maximal 30 Gew%.

Liegt eine der antimikrobiellen Substanzen in einem organischen Lösemittel gelöst vor, bezieht sich die oben genannte Einsatzkonzentration auf die reine Wirksubstanz, nicht auf das Lösemittel. Letzteres ist dem Lösungsmittelsystem (b) zuzuordnen.

Lösemittelphase (b):
Das Lösemittelsystem (b) der erfindungsgemäßen Zusammensetzung dient in erster Linie der Lösung der Komponente(n) (a), soweit diese nicht selbst als Lösung in einem organischen Lösemittel, bevorzugt einem Lösemittel gemäß (b) oder in einem Öl oder Lipid gemäß (d) oder als organische Flüssigkeit vorliegen, sowie der Komponente(n) (c), um eine einphasige, also homogene Mischung aller Komponenten zu gewährleisten. Das Lösemittelsystem kann daher eine oder mehrere Komponenten umfassen, die wiederum selbst mit dem Öl-/ Lipidsystem (d) eine homogene Mischung ergeben. Diese Komponenten sind bevorzugt ausgewählt aus kurzkettigen Alkoholen mit 2 bis 6 Kohlenstoffatomen sowie deren Isomeren, wie Ethanol, Propanol, n-Butanol, Isobutanol, n-Pentanol, Isopentanol, n- Hexanol und Isohexanol, oder kurzkettigen Kohlenwasserstoffen mit 5 oder 6 Kohlenstoffatomen sowie deren Isomere, wie n-Pentan, Isopentan, n-Hexan oder Isohexan. Ebenso könnten Glycole, wie Monopropylenglycol, Dipropylenglycol und Glycolether, wie Dipropylenglykol-mono-methylether, 3-Butoxypropan-2-ol in dieser Komponente enthalten sein. Ein besonders bevorzugtes Lösemittel ist Isopentan. Ein weiteres bevorzugtes Lösemittel ist Ethanol.

Das Lösungsmittelsystem stellt 0 bis 70 Gew% der kosmetischen Zusammensetzung dar, bevorzugt 15 bis 60 Gew% und insbesondere bevorzugt 30 bis 50 Gew%. Im Falle, dass Ethanol als Lösemittel dient, ist der Einsatz dieser Komponente bevorzugt auf maximal 40 Gew% beschränkt, wobei das Lösungsmittelsystem neben dem Ethanol weitere der hier genannten Komponenten enthalten kann.

Hierbei ist zu beachten, dass die Komponenten teilweise neben ihrer Wirkung als Lösemittel auch selbst eine antimikrobielle Wirkung aufweisen, beispielsweise Ethanol. In diesem Falle sind sie einsetzbaren Mengen der Systeme (a) und (b) nicht kombinierbar, vielmehr ist beispielsweise der Einsatz von Ethanol auf den angegebenen Mengenbereich des Lösemittelsystems beschränkt, auch wenn hier eine zusätzliche Wirkung als antimikrobielles Mittel erzielt wird. Wird Ethanol als Lösemittel eingesetzt, wird bevorzugt wenigstens ein zusätzlicher antimikrobieller Wirkstoff aus der oben beschriebenen Gruppe (a) eingesetzt.

In einer bevorzugten Ausführungsform enthält die Zusammensetzung als einziges Lösemittel Isopentan. In einer weiteren Ausführungsform enthält die Zusammensetzung als einziges Lösemittel Ethanol. In der vorliegenden Zusammensetzung bewirkt der Einsatz von Ethanol nicht wie in kosmetischen Zusammensetzungen auf Alkoholbasis eine Austrocknung der Haut, weil die Zusammensetzung außer dem Lösemittelsystem einen hohen Lipid- und / oder Ölanteil aufweist.

Kosmetische Hilfs- und Zusatzstoffe (c):
Die kosmetische Zusammensetzung kann in kosmetischen Mitteln übliche Hilfs- und Zusatzstoffe enthalten, soweit sie in den Phasen (b) und / oder (d) löslich oder mit diesen mischbar sind. Als übliche Zusatzstoffe werden hier insbesondere Parfums, bevorzugt Parfümöle, ätherische Öle oder Duftöle, aber auch Essenzen betrachtet. Dem Einsatz dieser Öle/Essenzen ist lediglich in Bezug auf die gewünschte Duftnote eine Grenze gesetzt, so dass jedes auf dem Markt befindliche Öl oder jede Essenz dieser Art in die erfindungsgemäße Zusammensetzung eingearbeitet werden kann.

Darüber hinaus können mit einem Gehalt von insgesamt 0-10 Gew% beispielsweise Vitamine und deren Derivate, z.B. Vitamin E Acetat und Extrakte aus Pflanzen und / oder Früchten sowie UV-Filter eingearbeitet werden.

Öl-/ Lipidphase (d):
In der Öl- / Lipidphase (d) der kosmetischen Zusammensetzung können Öle oder Lipide oder Öle und Lipide eingesetzt werden. Hierbei kann als Ölphase ein einzelnes Öl oder ein Gemisch von Ölen verwendet werden, als Lipidphase kann ein einzelner Lipidtyp oder ein Gemisch verschiedener Lipidtypen eingesetzt werden.

Die Ölphase der erfindungsgemäßen Formulierungen kann gewählt sein aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 30, bevorzugt 8 bis 24, insbesondere 12 bis 18 C-Atomen. Die Fettsäuretriglyceride können beispielsweise ausgewählt werden aus der Gruppe der synthetischen, halbsynthetischen und natürlichen pflanzlichen Öle.

Als pflanzliche Öle sind beispielsweise geeignet: Aloe vera Öl, Aprikosenkemöl, Avocadoöl. Baobaböl, Bienenwachs, Calendulaöl, Distelöl, Erdnussöl, Hanföl, Jojobaöl, Kamillenöl, Kokosnussfett, Kokosöl, Kürbiskernöl, Leinöl, Macadamiaöl, Maisöl, Mandelöl (süss), Mohnöl, Nachtkerzenöl, Olivenöl, Palmkernöl, Palmöl, Pfirsichkernöl, Rapsöl, Sanddornöl, Sesamöl, Sheabutter, Sojaöl, Sonnenblumenöl, Traubenkemöl, Walnussöl, Weizenkeimöl, Wildrosenöl und ähnliche. Bevorzugt enthält die Zusammensetzung süßes Mandelöl, Nachtkerzenöl, Olivenöl oder Jojobaöl, besonders bevorzugt Mandelöl oder Nachtkerzenöl.

Weitere polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können gewählt werden aus der Gruppe Octylpalmitat, Octylcocoat, Octylisostearat, Octyldodeceylmyristat, Cetearylisononanoat, Isopropylmyristat, Isopropylpalmitat, Isopropylstearat, Isopropyloleat, n-Butylstearat, n-Hexyllaurat, n-Decyl-oleat, Isooctylstearat, Isononylstearat, Isononylisononanoat, 2-Ethylhexylpalmitat, 2-Ethylhexyllaurat, 2-Hexyldecylstearat, 2-Octyidodecylpalmitat, Stearylheptanoat, Oleyloleat, Oleylerucat, Erucyloleat, Erucylerucat, Tridecylstearat, Tridecyltrimeilitat, sowie synthetische, halbsynthetische und natürliche Gemische solcher Ester, wie z. B, das oben bereits genannte Jojobaöl.

Ferner kann die Ölphase vorteilhaft gewählt werden aus der Gruppe der Dialkylether und Dialkylcarbonate, vorteilhaft sind z. B. Dicaprylylether (Cetiol OE) und/oder Dicaprylylcarbonat, beispielsweise das unter der Handelsbezeichnung Cetiol CC bei der Fa. Cognis erhältliche Öl.

Es ist außerdem möglich die Ölkomponente(n) aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, Cis-13-Alkyllactat, Di-Cis-is-Aikyitartrat, Triisostearin, Dipentaerythrityl Hexacaprylat/Hexacaprat, Propylenglykolmonoisostearat, Tricaprylin, Dimethylisosorbid zu wählen. Auch beliebige Abmischungen solcher Öl- und Wachskomponenten können im Sinne der vorliegenden Erfindung eingesetzt werden.

Ferner kann die Ölphase auch unpolare Öle enthalten, beispielsweise solche aus der Gruppe der verzweigten und unverzweigten Kohlenwasserstoffe, insbesondere Mineralöl, Vaseline (Petrolatum), Paraffinöl, Squalan und Squalen, Polyolefine, hydrogenierte Polyisobutene und Isohexadecan, sowie polymerisierte Silicone (z.B. Belsil^{™} von der Firma Wacker Silicones) Unter den Polyolefinen sind Polydecene die bevorzugten Substanzen.

Lipide:
Die Öl-/ Lipidphase (d) kann neben oder anstelle der oben genannten Ölkomponenten auch wenigstens ein Lipid oder zwei, drei oder mehrere Lipide umfassen oder aus diesen bestehen. Hierbei können die Lipide ausgewählt sein aus der Gruppe der natürlichen und/oder synthetischen Lipide, der Fettsäuren, der verzweigten und unverzweigten langkettigen Alkohole (Fettalkohole) mit C₈ bis C₃₀, Ester von Fettsäuren mit Alkoholen oder von Fettalkoholen mit Säuren oder der flüssigen Wachse.

Fettsäuren:
Hexansäure, Heptansäure, Octansäure, Nonansäure, Decansäure, Dodecansäure, Tetradecansäure, Hexadecansäure, Heptadecansäure, Octadecansäure, Eicosan-/Icosansäure, Docosansäure, Tetracosansäure, Hexacosansäure, Octaacosansäure, Triacontansäure. Die entsprechenden Trivialnamen lauten Capronsäure, Önanthsäure, Caprylsäure, Pelargonsäure, Caprinsäure, Laurinsäure, Myristinsäure, Palmitinsäure, Margarinsäure, Stearinsäure, Arachinsäure, Behensäure, Lignocerinsäure, Cerotinsäure, Montansäure, Melissinsäure. Ebenfalls geeignet sind Undecylensäure, Myristoleinsäure, Palmitoleinsäure, Petroselinsäure, Ölsäure, Elaidinsäure, Vaccensäure, Gadoleinsäure, Icosensäure, Cetoleinsäure, Erucasäure, Nervonsäure, Linolsäure, Alpha-Linolensäure, Gamma-Linolensäure, Calendulasäure, Punicinsäure, Alpha-Eleostearinsäure, Arachidonsäure, Timnodonsäure, Clupanodonsäure, Cervonsäure, Vernolsäure und Rizinolsäure.

Fettalkohole:
Geeignete Fettalkohole sind beispielsweise gesättigte oder ungesättigte Fettalkohole mit 8 bis 30 Kohlenstoffatomen, wie Octanol, Decanol, Dodecanol (Laurylalkohol), Tetradecanol (Myristylalkohol), Hexadecanol(Cetylalkohol), Heptadecanol, Octadecanol (Stearylalkohol), Octyldodecanol, Eicosanol, Behenylalkohol, n-Docosanol, oder Delta-9-cis-Hexadecenol, Delta-9-Octadecenol, trans-Delta-9-Octadecenol, cis-Delta-11-Octadecenol oder Octadecatrienol.

Ester von Fettsäuren mit Alkoholen oder von Fettalkoholen mit Säuren:
Geeignete Ester sind solche aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Ester können gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, Glycerylmonostearat, Isostearyl-isostearat, Glyceryltriacetate, Propylenglycol Monolaurat, Menthyllactat, Isostearylpalmitat, Myristylmyristat, C12-15 Alkylbenzoat; Benzylbenzoat, Diisooctyladipat, 2-Octyldodecanol, Propylenecarbonat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester oder flüssigen Wachse.

Des weiteren können Ester einer verzweigten oder unverzweigten aliphatischen C₃ bis C₁₀ Säure mit einem verzweigten oder unverzweigten aliphatischen C₁₆ bis C₂₄ Alkohol in die Lipidphase der Zusammensetzung eingesetzt werden. Bevorzugt sind hierbei Ester der Heptansäure oder der Octansäure mit Stearylalkohol, also Stearylheptanoat oder Stearyloctanoat.

Die Öl-/ Lipidphase (d) enthält bevorzugt wenigstens eine Komponente aus der Gruppe 2-Ethylhexylisostearat, Octyldodecanol, Isotridecylisononanoat, Isoeicosan, 2-Ethylhexylcocoat, C12-15-Alkylbenzoat, Capryl-Caprinsäure-Trlglycerid, Dicaprylylether, Dicaprylyl Carbonat, Butylen Gylcol und Dicaprylat/Dicaprat, Isopropylmyristat und Isopropylpalmitat.

Besonders vorteilhaft sind Mischungen aus C12-15-Alkylbenzoat und Isopropylmyristat, Mischungen aus C12-15-Alkylbenzoat und Isoproyplpalmitat sowie Mischungen aus C12-15-Alkylbenzoat, Isopropylmyristat und Isopropylpalmitat.

Ebenfalls besonders bevorzugt ist eine Mischung aus wenigstens einer Öl- und wenigstens einer Lipidkomponente, beispielsweise aus einem pflanzlichen Öl und C12-15 Alkylbenzoat und ggf. Capryl/Capric Triglycerin, oder aus einem pflanzlichen Öl, C12-15 Alkylbenzoat, Octyldodecanol und ggf. Capryl/Capric Triglycerin.

Die Öl- /Lipidphase (d) wird in einer Menge in die erfindungsgemäße Zusammensetzung eingesetzt, die der Restmenge ad. 100 Gew% der Zusammensetzung entspricht. Dies bedeutet, dass die Phasen (a), (b) und (c) die oben angegebenen Gew% der Zusammensetzung ausmachen und der verbleibende Rest der Zusammensetzung durch die Phase (d) dargestellt wird.

Da es sich bei der erfindungsgemäßen Zusammensetzung um ein homogenes Gemisch von organischen Phasen handelt, in denen gegebenenfalls organische Substanzen gelöst vorliegen, ist es nicht notwendig Emulgatoren zu verwenden und erfindungsgemäß auch bevorzugt, dass keine Emulgatoren in die Zusammensetzung eingearbeitet werden. Insbesondere enthält die Zusammensetzung kein Wasser bzw. keine wässrige Lösung.

Darüber hinaus ist es bevorzugt in das Gemisch keine Antitranspiranzien einzuarbeiten, insbesondere keine antitranspiranten Salze, beispielsweise Aluminiumsalze wie Aluminiumchlorhydrat, um die Bildung weißer Flecke auf Haut und Kleidung zu vermeiden.

Die Phasen (b) und (d), sowie die optionale Phase (c) können nahezu vollständig aus organischen, insbesondere öligen oder lipidhaltigen Komponenten bestehen. In diesem Zusammenhang bedeutet "nahezu vollständig", dass diese Phasen zu mehr als 90%, bevorzugt mehr als 95%, weiter bevorzugt zu mehr als 98% und insbesondere bevorzugt zu wenigstens 99% aus organischen, insbesondere öligen oder lipidhaltigen Komponenten bestehen, wobei in diesem Falle Isopentan den öligen Komponenten zuzuordnen ist. Darüber hinaus stellt die Phase (a) der Zusammensetzung eine in den Phasen (b) und/oder (d) vollständig lösliche oder homogen mit diesen Phasen mischbare Phase dar. Somit kann die erfindungsgemäße Zusammensetzung insgesamt nahezu vollständig aus organischen, insbesondere aus öligen oder lipidhaltigen Komponenten bestehen. Der hohe Anteil der öligen und oder lipidhaltigen Phasen trägt zur hohen Pflegewirkung der Zusammensetzung der Haut bei, so dass ein Austrocknen der Haut auch bei regelmäßiger Anwendung vermieden wird.

Die kosmetische Zusammensetzung kann als ein durch Gas ausgetriebenes Spray aus einem unter Druck stehenden Behälter ("Sprayformulierung"), als Pumpspray, das beispielsweise mit Hilfe eines Triggers versprüht wird, als Roll-on Formulierung oder Dap-on Formulierung (wird mit einem Schwämmchen aufgetragen), oder als Körperöl breitgestellt werden. Im letzteren Falle wird die Kosmetische Zusammensetzung beispielsweise in einer Flasche angeboten, aus der die Zusammensetzung durch ledigliches Kippen der Falsche entnommen werden kann.

Bei der Bereitstellung der kosmetischen Zusammensetzung als Pumpspray, Roll-on Formulierung oder als Körperöl wird diese einfach in einen entsprechenden Behälter gefüllt, ohne dass weitere Zusätze zu der Formulierung nötig wären.

Bei der Bereitstellung als Sprayformulierung kann die Zusammensetzung entweder ohne weitere Zusätze in einen Behälter gefüllt werden, der unter Druck stabil bleibt, und anschließend wird dem Behälter ein Gas zugesetzt, das das Austreiben der Formulierung aus dem Behälter zu einem späteren Zeitpunkt ermöglicht, oder die Zusammensetzung wird mit einem Gas oder einer Gasmischung versetzt, bevor sie in einen geeigneten Behälter gefüllt wird. Hierbei ist der Zusatz des Gases in die Zusammensetzung bevorzugt.

Die Zusammensetzung kann beispielsweise mit einem komprimierten oder unter Druck verflüssigten Gas der Gruppe Propan, n-Butan, iso-Butan oder Dimethylether (DME) versetzt werden, bevorzugt mit einem Gemisch aus n-Butan und Propan. Diese Gase, insbesondere die beiden letztgenannten Gase können in sämtlichen / beliebigen Mischungsverhältnissen eingesetzt werden.

Ein solches Gasgemisch kann beispielsweise unter Druck vollständig in die Zusammensetzung eingearbeitet werden. Das gesamte Gemisch, also die kosmetische Zusammensetzung einschließlich des Gases liegt als homogene flüssige Phase und Dampfdruckphase des Treibmittels vor.

Der Anteil der Gasphase an dem so hergestellten Gemisch kann bis zur Gassättigung reichen, bevorzugt wird eine Gaskonzentration von bis zu 90,0% eingestellt.

Da sich alle Bestandteile der kosmetischen Zusammensetzung ineinander lösen oder homogen miteinander mischbar sind, ist die Herstellungsweise der kosmetischen Zusammensetzung denkbar einfach. Die einzelnen Komponenten müssen lediglich zusammengegeben werden und miteinander vermischt werden, ohne dass eine Dispersion oder Emulsion der Bestandteile notwendig wird. Somit ist auch keine aufwendige Ausstattung mit Maschinen für die Herstellung der erfindungsgemäßen Produktes notwendig, da das Produkt weder als Emulsion, noch als Suspension oder Dispersion hergestellt wird. Das Produkt stellt vielmehr eine stabile Lösung der Komponenten dar, woraus sich eine einphasige klare bis opaleszente Lösung ergibt, die auch nicht vor Gebrauch als Körperpflegemittel geschüttelt zu werden braucht.

Die erfindungsgemäße kosmetische Zusammensetzung kann in erster Linie zur Verringerung der Entwicklung eines unangenehmen Körpergeruches eingesetzt werden. Bevorzugt wird die Zusammensetzung als Deodorant, Körperspray, Körper-oder Gesichtspflegemittel, oder Make-up Entfernungsmittel, Massageöl verwendet.

Beispiel:
Die folgende Tabelle gibt beispielhafte Formulierungen der kosmetischen Zusammensetzung wieder, ohne dass die Erfindung auf die spezifische Kombination und Einsatzmengen der Wirkstoffe beschränkt sein soll.

| **Formulierung** | **1** | **2** | **3** | **4** | **5** |
|---|---|---|---|---|---|
| **Antimikrobieller Wirkstoff** | | | | | |
| Phenoxyethanol | 5-10% | 5-10% | | 10-18% | 5-10% |
| Ethylhexylglycerin | 2-8% | 2-8% | 2-8% | | |
| Benzisothiazolin-on | | | 5-15% | | 3-8% |
| | | | | | |
| **Lösemittel** | | | | | |
| Ethanol | 20-40% | | | 20-40% | |
| Isopentan | | 25-50% | | | 25-50% |
| | | | | | |
| **Zusatzmittel** | | | | | |
| Parfümöl | 0,5-8% | | | | 0,2- 6% |
| Vitamin E Acetat | | | 1-5% | | |
| | | | | | |
| **Öl-/ Lipidsystem** | | | | | |
| C12-15 Alkylbenzoat | 5-15% | 5-15% | 5-15% | 5-15% | |
| Octyldodecanol | 5-15% | | | 5-15% | 5-15% |
| Pflanzliches Öl | Mandelöl 0,2-1 % | Nachtkerzenöl 5-15%, Mandelöl 0,2-1% | | Jojobaöl 0,5 bis 2% | Macadamia-Öl 0,5-15% |
| **Caprylic/Capric Triglyceride** | Ad 100% | Ad 100% | Ad 100% | Ad 100% | Ad 100% |

## Patentansprüche

1. Kosmetische Zusammensetzung in Form eines homogenen Gemisches, umfassend
(a) 0,01 - 30 % ein antimikrobielles Wirksystem,
(b) 0 - 70 % ein Lösungsmittelsystem für (a) und/oder (c), und/oder (d)
(c) optional wenigstens einen üblichen Hilfs- oder Zusatzstoff, soweit sie in (a), (b) und/ oder (c) löslich oder mit diesen mischbar sind, und
(d) ad 100% ein Öl-/ Lipidsystem.

2. Kosmetische Zusammensetzung gemäß Anspruch 1, **dadurch gekennzeichnet, dass** das antimikrobielle Wirksystem (a) wenigstens einen Wirkstoff umfasst, der auf Bakterien, antibakteriell wirkt.

3. Kosmetische Zusammensetzung gemäß Anspruch 2, **dadurch gekennzeichnet, dass** der Wirkstoff ausgewählt ist aus bakteriostatischen oder bakterioziden Wirkstoffen oder einer Mischung daraus.

4. Kosmetische Zusammensetzung gemäß einem der Ansprüche 1 bis 3, **dadurch gekennzeichnet, dass** eine Mischung aus Lösungsmittelsystem (b) mit wenigstens einem Lösemittel mit dem Öl-/ Lipidsystem (d) eine homogene Mischung darstellt.

5. Kosmetische Zusammensetzung gemäß Anspruch 4, **dadurch gekennzeichnet, dass** das Lösungsmittelsystem wenigstens ein Lösungsmittel, ausgewählt aus der Gruppe der kurzkettigen Alkohole mit 2 bis 6 Kohlenstoffatomen sowie deren Isomeren, wie Ethanol, Propanol, n-Butanol, Isobutanol, n-Pentanol, Isopentanol,n- Hexanol, Isohexanol, oder der kurzkettigen Kohlenwasserstoffe mit 5 oder 6 Kohlenstoffatomen sowie deren Isomeren, wie n-Pentan, Isopentan, n-Hexan oder Isohexan, umfasst.

6. Kosmetische Zusammensetzung gemäß einem der Ansprüche 1 bis 5, **dadurch gekennzeichnet, dass** der/die Hilfs- und Zusatzstoffe (c) ausgewählt ist/sind aus der Gruppe bestehend aus Parfüm, Parfümöl, ätherischem Öl, Duftöl, Vitamine und deren Derivate, Extrakte aus Pflanzen und Früchten, UV-Filter.

7. Kosmetische Zusammensetzung gemäß einem der Ansprüche 1 bis 6, **dadurch gekennzeichnet, dass** das Öl-/ Lipidsystem (d) wenigstens eine Komponente umfasst, ausgewählt aus der Gruppe der Lipide, Triglyceride, Fettsäuren, verzweigten und unverzweigten langkettigen Alkohole mit C₈ bis C₃₀, Ester von Fettsäuren mit Alkoholen oder von Fettalkoholen mit Säuren, pflanzlichen Öle, Paraffinöl, Lanolin, Sheabutter.

8. Kosmetische Zusammensetzung gemäß einem der Ansprüche 1 bis 7, **dadurch gekennzeichnet, dass** sie in Form einer Sprayformulierung, eines Pumpsprays, einer Roll-on Applikation, einer Dap-on Applikation oder eines Körperöls vorliegt.

9. Kosmetische Zusammensetzung gemäß einem der Ansprüche 1 bis 8, **dadurch gekennzeichnet, dass** die Zusammensetzung keine(n) Emulgator(en) enthält.

10. Kosmetische Zusammensetzung gemäß einem der Ansprüche 1 bis 9, **dadurch gekennzeichnet, dass** die Zusammensetzung keinen schweißhemmenden Wirkstoff (Antiperspirant), insbesondere kein Aluminiumsalz wie Aluminiumchlohydrat enthält.

11. Kosmetische Zusammensetzung gemäß einem der Ansprüche 1 bis 10, **dadurch gekennzeichnet, dass** die Zusammensetzung eine einphasige klare bis opaleszente Lösung darstellt.

12. Verwendung einer Zusammensetzung gemäß einem der Ansprüche 1 bis 11 zur Verhinderung oder Verringerung der Entwicklung von Körpergeruch.

13. Verwendung gemäß Anspruch 12 als Deodorant, Körperspray, Körper- oder Gesichtspflegemittel, oder Make-up Entfernungsmittel, oder Anti-aging Pflegemittel.
